(19) Europäisches Patentamt / European Patent Office / Office européen des brevets

(11) **EP 3 896 097 A1**

(12) **EUROPEAN PATENT APPLICATION**
published in accordance with Art. 153(4) EPC

(43) Date of publication:
**20.10.2021 Bulletin 2021/42**

(21) Application number: **19896711.9**

(22) Date of filing: **12.12.2019**

(51) Int Cl.:
*C08F 20/06* (2006.01)    *C08J 3/12* (2006.01)
*A61F 13/53* (2006.01)    *B01J 20/26* (2006.01)
*B01J 20/28* (2006.01)

(86) International application number:
**PCT/JP2019/048820**

(87) International publication number:
**WO 2020/122217 (18.06.2020 Gazette 2020/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 12.12.2018  JP 2018232724
12.12.2018  JP 2018232843
12.12.2018  JP 2018232847
12.12.2018  JP 2018232726
12.12.2018  JP 2018232728
12.12.2018  JP 2018232848
12.12.2018  JP 2018232850
12.12.2018  JP 2018232851
12.12.2018  JP 2018232856
12.12.2018  JP 2018232857
30.01.2019  JP 2019014528
22.03.2019  JP 2019055289

(71) Applicant: **Sumitomo Seika Chemicals Co., Ltd.**
**Kako-gun, Hyogo 675-0145 (JP)**

(72) Inventor: **HAMA Maoki**
**Himeji-shi, Hyogo 672-8076 (JP)**

(74) Representative: **Nederlandsch Octrooibureau**
**P.O. Box 29720**
**2502 LS The Hague (NL)**

(54) **WATER-ABSORPTIVE RESIN PARTICLE, ABSORPTION BODY, AND ABSORPTIVE ARTICLE**

(57)    An absorbent article 100 includes an absorber 10, the absorber 10 contains water-absorbent resin particles 10a, and in the water-absorbent resin particles 10a, a water absorption rate based on the Vortex method at 300 rpm is 10 to 50 seconds.

*Fig.1*

## Description

### Technical Field

The present invention relates to water-absorbent resin particles, an absorber, and an absorbent article.

### Background Art

In the related art, an absorber containing water-absorbent resin particles has been used in an absorbent article for absorbing a liquid (for example, urine) having water as a main component. For example, Patent Literatures 1 and 2 below disclose water-absorbent resin particles having a predetermined water absorption rate based on the Vortex method at 600 rpm in the related art.

### Citation List

### Patent Literature

[Patent Literature 1] Japanese Unexamined Patent Publication No. 2013-132433
[Patent Literature 2] Japanese Unexamined Patent Publication No. 2008-178667

### Summary of Invention

### Technical Problem

In a case where a liquid provided to an absorbent article does not sufficiently permeate the absorbent article, there may occur a problem that the excess liquid leaks to the outside of the absorbent article, for example, the excess liquid flows on the surface thereof. Therefore, it is required that the liquid permeates the absorbent article at a better permeation rate.
An object of an aspect of the present invention is to provide water-absorbent resin particles capable of obtaining an absorbent article having a better permeation rate. In addition, an object of another aspect of the present invention is to provide an absorber and an absorbent article using the water-absorbent resin particles.

### Solution to Problem

The present inventor has found that, even in a case where the water-absorbent resin particles are better in water absorption rate based on the Vortex method at 600 rpm in the related art, when the water-absorbent resin particles are used in the absorbent article, there is a case where it is difficult to obtain a better permeation rate, and under the circumstance, the present inventor has found that the water-absorbent resin particles having a suitable water absorption rate based on the low-speed flow Vortex method at 300 rpm are effective in obtaining an absorbent article having a better permeation rate.
An aspect of the present invention provides water-absorbent resin particles having a water absorption rate of 10 to 50 seconds based on the Vortex method at 300 rpm.
According to the above-mentioned water-absorbent resin particles, it is possible to obtain an absorbent article having a better permeation rate.
Another aspect of the present invention provides an absorber containing the above-mentioned water-absorbent resin particles.
Another aspect of the present invention provides a water-absorbent article including the above-mentioned absorber.

### Advantageous Effects of Invention

According to an aspect of the present invention, it is possible to provide water-absorbent resin particles capable of obtaining an absorbent article having a better permeation rate. In addition, according to another aspect of the present invention, it is possible to provide an absorber and an absorbent article using the water-absorbent resin particles. According to another aspect of the present invention, it is possible to provide use of a resin particle, an absorber, and an absorbent article to the absorption of a liquid.

**Brief Description of Drawings**

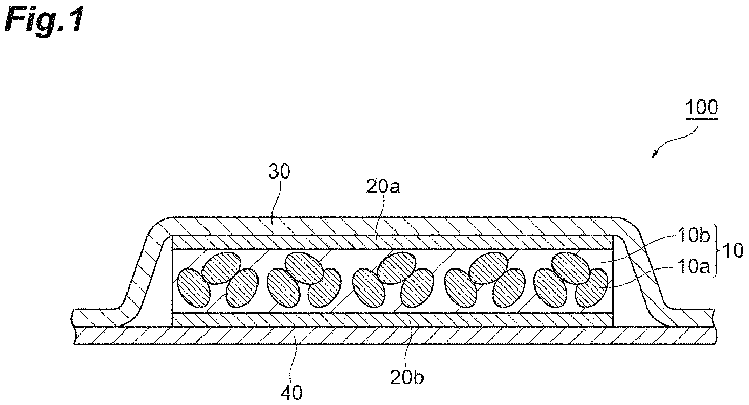

Fig. 1 is a cross-sectional view showing an example of an absorbent article.

**Description of Embodiments**

Hereinafter, embodiments of the present invention will be described in detail. However, the present invention is not limited to the following embodiments, and can be variously modified and implemented within the scope of the gist thereof.

In the present specification, "acrylic" and "methacryl" are collectively referred to as "(meth)acrylic". Similarly, "acrylate" and "methacrylate" are also referred to as "(meth)acrylate". In a numerical value range described in stages in the present specification, an upper limit value or a lower limit value of the numerical value range of a stage can be optionally combined with the upper limit value or the lower limit value of the numerical value range of another stage. In a numerical value range described in the present specification, the upper limit value or the lower limit value of the numerical value range may be replaced with the value shown in the examples. "Water-soluble" means that it exhibits a solubility in water of 5% by mass or more at 25°C. Materials exemplified in the present specification may be used alone, or may be used in combination of two or more. The content of each component in the composition means the total amount of a plurality of substances present in the composition in a case where the plurality of substances corresponding to each component are present in the composition, unless otherwise specified. "Physiological saline" refers to 0.9% by mass sodium chloride aqueous solution.

In the water-absorbent resin particles of the present embodiment, the water absorption rate based on the Vortex method at the rotation speed of 300 rpm (rpm = min$^{-1}$) (low-speed flow Vortex method) is 10 to 50 seconds. According to such water-absorbent resin particles, it is possible to obtain an absorbent article having a better permeation rate. In addition, according to the water-absorbent resin particles of the present embodiment, when an absorbent member is brought into contact with a liquid supply position of the absorbent article in a state where the liquid has permeated the absorbent article, the liquid that has permeated the absorbent article is not easily absorbed in the absorbent member (that is, better quick-drying property is obtained).

The water absorption rate based on the Vortex method at 600 rpm in the related art, which was established by JIS in 1996, has been useful as a simple index that can easily compare resins produced by various production methods in the water-absorbent resin industry where technological development has been diverse. However, as the production method is further evolved, or the water-absorbent characteristics are imparted by various modifiers, there occur cases where this index is not sufficient for applications such as absorbent articles (for example, diapers).

In this regard, the present inventors presumed that the Vortex method not only measures the rapidity of contact and uptake of a liquid and a resin, but also comprehensively evaluates properties such as the easiness of block formation by the shape, and gel strength (easiness of swelling) under the circumstance where a certain degree or more of absorption capacity of the resin is needed in order to converge the vortex generated by the flow of the liquid. Then, a hypothesis that, in recent water-absorbent resins, there is a room for improvement for the degree of dynamic load as an index in the absorbent article has been reached, and a low-speed flow Vortex method at 300 rpm has been found.

The water absorption rate based on the low-speed flow Vortex method at 300 rpm (Vortex method of 300 rpm) can be obtained based on the Vortex method according to the Japanese Industrial Standard JIS K 7224 (1996) also disclosed in Patent Literatures 1 and 2 above, except that the rotation speed of the Vortex method in the related art is changed from 600 rpm to 300 rpm. As the water absorption rate based on the low-speed flow Vortex method at 300 rpm, the water absorption rate at 25°C can be used. Specifically, it is possible to obtain the water absorption rate as the time [second] from after the addition of the water-absorbent resin particles until the vortex disappears and the liquid surface becomes flat, when 2.0 ± 0.002 g of the water-absorbent resin particles are added to 50 ± 0.1 g of physiological saline stirred at 300 rpm. The water absorption rate based on the low-speed flow Vortex method at 300 rpm is the water absorption rate of physiological saline based on the Vortex method at 300 rpm.

The water absorption rate based on the low-speed flow Vortex method at 300 rpm is preferably 48 seconds or less, 45 seconds or less, 42 seconds or less, or 41 seconds or less from a viewpoint of easily obtaining better permeation rate and quick-drying property. The water absorption rate based on the low-speed flow Vortex method may be 40 seconds or less, 39 seconds or less, 38 seconds or less, 37 seconds or less, 36 seconds or less, 35 seconds or less, 34 seconds or less, 33 seconds or less, or 32 seconds or less. The water absorption rate based on the low-speed flow Vortex method is preferably 15 seconds or more, 20 seconds or more, 25 seconds or more, or 30 seconds or more from a viewpoint of easily avoiding gel blocking due to excessively rapid absorption. The water absorption rate based on the low-speed flow Vortex method may be 32 seconds or more, 34 seconds or more, 35 seconds or more, or 37 seconds or more. The water absorption rate based on the low-speed flow Vortex method may be 30 to 50 seconds, 35 to 50 seconds, 35 to 50 seconds, 37 to 50 seconds, 37 to 48 seconds, 37 to 45 seconds, or 37 to 42 seconds.

In the water-absorbent resin particles of the present embodiment, the water absorption rate based on the Vortex method at 600 rpm in the related art (Vortex method at 600 rpm) may be in the following range. The water absorption rate based on the Vortex method in the related art may be 60 seconds or less, 55 seconds or less, 50 seconds or less, 48 seconds or less, 47 seconds or less, 46 seconds or less, or 45 seconds or less. The water absorption rate based on the Vortex method in the related art may be 10 seconds or more, 15 seconds or more, 20 seconds or more, 25 seconds or more, 30 seconds or more, 32 seconds or more, 34 seconds or more, 36 seconds or more, 38 seconds or more, 40 seconds or more, 42 seconds or more, or 43 seconds or more. From these viewpoints, the water absorption rate based on the vortex method in the related art may be 10 to 60 seconds, 20 to 60 seconds, 30 to 60 seconds, 40 to 60 seconds, 40 to 55 seconds, 40 to 47 seconds, or 40 to 45 seconds. The water absorption rate based on the Vortex method in the related art can be obtained based on the Vortex method according to the Japanese Industrial Standard JIS K 7224 (1996). As the water absorption rate based on the Vortex method in the related art, the water absorption rate at 25°C can be used. The water absorption rate based on the Vortex method at 600 rpm in the related art is the water absorption rate of physiological saline based on the Vortex method at 600 rpm.

The ratio $R_{sf}/R_c$ of the water absorption rate $R_{slow\ fluid}$ ($R_{sf}$) based on the low-speed flow Vortex method with respect to the water absorption rate $R_{conventional}$ ($R_c$) based on the Vortex method in the related art is preferably 1.30 or less, 1.29 or less, 1.25 or less, 1.20 or less, 1.15 or less, 1.10 or less, or 1.09 or less from a viewpoint of easily obtaining better permeation rate and quick-drying property. The ratio $R_{sf}/R_c$ is preferably 0.50 or more, 0.60 or more, 0.70 or more, or 0.75 or more from a viewpoint of easily avoiding gel blocking due to excessively rapid absorption. From these viewpoints, the ratio $R_{sf}/R_c$ is preferably 0.50 to 1.30. The ratio $R_{sf}/R_c$ may be 1.08 or less, 1.05 or less, 1.00 or less, 0.95 or less, 0.93 or less, or 0.92 or less. The ratio $R_{sf}/R_c$ may be 0.80 or more, 0.85 or more, or 0.90 or more.

The water-absorbent resin particles of the present embodiment may be any water-absorbent resin particles as long as the water-absorbent resin particles can retain water, and the liquid to be absorbed can contain water. The water-absorbent resin particles of the present embodiment are better in absorbency of a body fluid such as urine, sweat, blood (for example, menstrual blood). The water-absorbent resin particles of the present embodiment can be used as a constituent component of the absorber of the present embodiment.

The water retention amount of physiological saline of the water-absorbent resin particles of the present embodiment is preferably in the following range. The water retention amount is preferably 10 g/g or more, 15 g/g or more, 20 g/g or more, 25 g/g or more, or 30 g/g or more from a viewpoint of easily obtaining better permeation rate and quick-drying property. The water retention amount is preferably 80 g/g or less, 75 g/g or less, 70 g/g or less, 65 g/g or less, 60 g/g or less, 55 g/g or less, 50 g/g or less, 48 g/g or less, or 45 g/g or less from a viewpoint of easily obtaining better permeation rate and quick-drying property. From these viewpoints, the water retention amount is preferably 10 to 80 g/g. The water retention amount may be 32 g/g or more or 34 g/g or more. The water retention amount may be 43 g/g or less, 42 g/g or less, 40 g/g or less, or 39 g/g or less. The water retention amount may be 20 to 80 g/g, 30 to 80 g/g, 32 to 80 g/g, 34 to 80 g/g, 34 to 75 g/g, 34 to 70 g/g, 20 to 60 g/g, 30 to 60 g/g, 30 to 50 g/g, 30 to 45 g/g, or 30 to 40 g/g. As the water retention amount, a water retention amount at room temperature (25 ± 2°C) can be used. The water retention amount can be measured by the method described in examples to be described later.

The bulk density of the water-absorbent resin particles of the present embodiment may be 0.58 g/mL or more, 0.59 g/mL or more, 0.60 g/mL or more, 0.61 g/mL or more, 0.62 g/mL or more, more than 0.62 g/mL, 0.63 g/mL or more, 0.64 g/mL or more, 0.65 g/mL or more, or 0.66 g/mL or more. The bulk density may be 0.90 g/mL or less, 0.88 g/mL or less, 0.86 g/mL or less, 0.84 g/mL or less, 0.82 g/mL or less, or 0.80 g/mL or less. The bulk density can be measured according to JIS K6219-2 (2005). The measurement is performed 5 times (n = 5), each value of the highest point and the lowest point is deleted, and an average value of the remaining 3 points can be obtained as the measurement value. The measurement is performed at 23 ± 2°C and the relative humidity of 50 ± 5%, and the measurement can be performed after the sample is stored in the same environment for 24 hours or more before the measurement.

Examples of the shape of the water-absorbent resin particles of the present embodiment include substantially spherical, crushed, and granular shapes. The medium particle diameter of the water-absorbent resin particles of the present embodiment may be 250 to 850 μm, 300 to 700 μm, or 300 to 600 μm. The water-absorbent resin particles of the present embodiment may have a desired particle size distribution at the time of being obtained by a production method to be described later, but the particle size distribution may be adjusted by performing an operation such as particle size adjustment using classification with a sieve.

The water-absorbent resin particles of the present embodiment can contain a crosslinking polymer (crosslinking polymer having a structural unit derived from an ethylenically unsaturated monomer) obtained by polymerizing a monomer containing an ethylenically unsaturated monomer, as polymer particles, for example. That is, the water-absorbent resin particles of the present embodiment can have a structural unit derived from an ethylenically unsaturated monomer. As the ethylenically unsaturated monomer, a water-soluble ethylenically unsaturated monomer can be used. Examples of the polymerization method include a reverse phase suspension polymerization method, an aqueous solution polymerization method, a bulk polymerization method, and a precipitation polymerization method. Among these, the reverse phase suspension polymerization method or the aqueous solution polymerization method is preferable from a viewpoint of ensuring good water-absorbent characteristics (water absorption rate and the like) of the obtained water-absorbent resin particles and facilitating control of the polymerization reaction. In the following, as a method for polymerizing an ethylenically unsaturated monomer, a reverse phase suspension polymerization method will be described as an example.

The ethylenically unsaturated monomer is preferably water-soluble, and examples thereof include (meth)acrylic acid and a salt thereof, 2-(meth)acrylamide-2-methylpropanesulfonic acid and a salt thereof, (meth)acrylamide, N, N-dimethyl (meth)acrylamide, 2-hydroxyethyl (meth)acrylate, N-methylol (meth)acrylamide, polyethylene glycol mono(meth)acrylate, N, N-diethylaminoethyl (meth)acrylate, N, N-diethylaminopropyl (meth)acrylate, and diethylaminopropyl (meth)acrylamide. In a case where the ethylenically unsaturated monomer has an amino group, the amino group may be quaternized. The ethylenically unsaturated monomer may be used alone, or may be used in combination of two or more. Functional groups such as a carboxyl group and an amino group of the above-mentioned monomers can function as functional groups capable of crosslinking in a surface crosslinking step to be described later.

Among these, from a viewpoint of industrial availability, the ethylenically unsaturated monomer preferably contains at least one compound selected from the group consisting of (meth)acrylic acid and a salt thereof, acrylamide, methacrylamide, and N, N-dimethylacrylamide, and more preferably contains at least one compound selected from the group consisting of (meth)acrylic acid and a salt thereof, and acrylamide. From a viewpoint of further enhancing the water-absorbent characteristics (water absorption rate, water retention amount, and the like), the ethylenically unsaturated monomer further more preferably contains at least one compound selected from the group consisting of (meth)acrylic acid and a salt thereof. That is, the water-absorbent resin particles preferably have a structural unit derived from at least one selected from the group consisting of (meth)acrylic acid and a salt thereof.

As the monomer for obtaining the water-absorbent resin particles, a monomer other than the above-mentioned ethylenically unsaturated monomer may be used. Such a monomer can be used by being mixed with an aqueous solution containing the above-mentioned ethylenically unsaturated monomer, for example. The use amount of the ethylenically unsaturated monomer is preferably 70 to 100 mol% with respect to a total amount of the monomer (the total amount of the monomer for obtaining the water-absorbent resin particles. For example, a total amount of the monomers that provide a structural unit of the crosslinking polymer. The same applies hereinafter). Among these, the ratio of (meth)acrylic acid and a salt thereof is more preferably 70 to 100 mol% with respect to the total amount of the monomers. "Ratio of (meth)acrylic acid and a salt thereof" means the ratio of the total amount of (meth)acrylic acid and a salt thereof.

According to the present embodiment, as an example of the water-absorbent resin particles, it is possible to provide water-absorbent resin particles containing a crosslinking polymer having a structural unit derived from an ethylenically unsaturated monomer, in which the ethylenically unsaturated monomer contains at least one compound selected from the group consisting of (meth)acrylic acid and a salt thereof, and the ratio of (meth)acrylic acid and a salt thereof is 70 to 100 mol% with respect to the total amount of the monomer for obtaining the water-absorbent resin particles (for example, the total amount of the monomer that provides a structural unit of the crosslinking polymer), and these water-absorbent resin particles may have an aspect that the water retention amount of physiological saline is 32 to 80 g/g, the water absorption rate of physiological saline based on the Vortex method at 300 rpm is 35 to 50 seconds, and the water absorption rate of physiological saline based on the Vortex method at 600 rpm is 40 to 60 seconds.

The ethylenically unsaturated monomer is usually preferably used as an aqueous solution. The concentration of the ethylenically unsaturated monomer in the aqueous solution containing the ethylenically unsaturated monomer (hereinafter, simply referred to as "monomer aqueous solution") is preferably 20% by mass or more and a saturated concentration or less, more preferably 25 to 70% by mass, and further more preferably 30 to 55% by mass. Examples of the water used in the aqueous solution include tap water, distilled water, and ion-exchanged water.

In a case where the ethylenically unsaturated monomer has an acid group, the monomer aqueous solution may be used by neutralizing the acid group with an alkaline neutralizing agent. The degree of neutralization of the ethylenically unsaturated monomer by the alkaline neutralizing agent is preferably 10 to 100 mol%, more preferably 50 to 90 mol%, and further more preferably 60 to 80 mol% of the acid group in the ethylenically unsaturated monomer, from a viewpoint of increasing an osmotic pressure of the obtained water-absorbent resin particles, and further increasing the water-absorbent characteristics (water retention amount, water absorption rate, and the like). Examples of the alkaline neutralizing agent include alkali metal salts such as sodium hydroxide, sodium carbonate, sodium hydrogen carbonate, potassium hydroxide, and potassium carbonate; and ammonia. The alkaline neutralizing agent may be used alone, or may be used in combination of two or more. The alkaline neutralizing agent may be used in the form of an aqueous solution to simplify the neutralization operation. Neutralization of the acid group of the ethylenically unsaturated monomer can be performed by adding an aqueous solution of sodium hydroxide, potassium hydroxide, or the like dropwise in the above-mentioned monomer aqueous solution and mixing therewith.

In a reverse phase suspension polymerization method, a monomer aqueous solution is dispersed in a hydrocarbon dispersion medium in the presence of a surfactant, and polymerization of the ethylenically unsaturated monomer can be performed using a radical polymerization initiator or the like. As the radical polymerization initiator, a water-soluble radical polymerization initiator can be used.

Examples of the surfactant include a nonionic surfactant, and an anionic surfactant. Examples of the nonionic surfactant include sorbitan fatty acid ester, (poly)glycerin fatty acid ester ("(poly)" means both of a case where there is a prefix of "poly" and a case where there is no prefix thereof. The same applies hereinafter), sucrose fatty acid ester, polyoxyethylene sorbitan fatty acid ester, polyoxyethylene glycerin fatty acid ester, sorbitol fatty acid ester, polyoxyethylene sorbitol fatty acid ester, polyoxyethylene alkyl ether, polyoxyethylene alkyl phenyl ether, polyoxyethylene castor oil, polyoxyethylene hydrogenated castor oil, alkylallyl formaldehyde condensed polyoxyethylene ether, polyoxyethylene polyoxypropylene block copolymer, polyoxyethylene polyoxypropyl alkyl ether, and polyethylene glycol fatty acid ester. Examples of the anionic surfactant include fatty acid salt, alkylbenzene sulfonate, alkylmethyl taurate, polyoxyethylene alkylphenyl ether sulfate, polyoxyethylene alkyl ether sulfonate, phosphate ester of polyoxyethylene alkyl ether, and phosphate ester of polyoxyethylene alkylallyl ether. The surfactant may be used alone, or may be used in combination of two or more.

From a viewpoint of a good state of the W/O type reverse phase suspension, easily obtaining water-absorbent resin particles having a suitable particle diameter, and industrial availability, the surfactant preferably contains at least one compound selected from the group consisting of a sorbitan fatty acid ester, a polyglycerin fatty acid ester, and a sucrose fatty acid ester. From a viewpoint of easily obtaining an appropriate particle size distribution of the water-absorbent resin particles, and from a viewpoint of easily improving the water-absorbent characteristics (water absorption rate, and the like) of the water-absorbent resin particles and the performance of the absorbent article using the same, the surfactant preferably contains sucrose fatty acid ester, and more preferably contains sucrose stearic acid ester.

The use amount of the surfactant is preferably 0.05 to 10 parts by mass, more preferably 0.08 to 5 parts by mass, and further more preferably 0.1 to 3 parts by mass with respect to 100 parts by mass of the monomer aqueous solution from a viewpoint of obtaining a sufficient effect on the use amount and economic efficiency.

In the reverse phase suspension polymerization, a polymeric dispersant may be used in combination with the above-mentioned surfactant. Examples of the polymeric dispersant include maleic anhydride-modified polyethylene, maleic anhydride-modified polypropylene, maleic anhydride-modified ethylene/propylene copolymer, maleic anhydride-modified EPDM (ethylene propylene diene terpolymer), maleic anhydride-modified polybutadiene, maleic anhydride/ethylene copolymer, maleic anhydride/propylene copolymer, maleic anhydride/ethylene/propylene copolymer, maleic anhydride/butadiene copolymer, polyethylene, polypropylene, ethylene/propylene copolymer, oxidized polyethylene, oxidized polypropylene, oxidized ethylene/propylene copolymer, ethylene/acrylic acid copolymer, ethyl cellulose, and ethyl hydroxyethyl cellulose. The polymeric dispersant may be used alone or may be used in combination of two or more. From a viewpoint of better dispersion stability of the monomer, the polymeric dispersant is preferably at least one selected from the group consisting of maleic anhydride-modified polyethylene, maleic anhydride-modified polypropylene, maleic anhydride-modified ethylene/propylene copolymer, maleic anhydride/ethylene copolymer, maleic anhydride/propylene copolymer, maleic anhydride/ethylene/propylene copolymer, polyethylene, polypropylene, ethylene/propylene copolymer, oxidized polyethylene, oxidized polypropylene, and oxidized ethylene/propylene copolymer.

The use amount of the polymeric dispersant is preferably 0.05 to 10 parts by mass, more preferably 0.08 to 5 parts by mass, and further more preferably 0.1 to 3 parts by mass with respect to 100 parts by mass of the monomer aqueous solution, from a viewpoint of obtaining a sufficient effect on the use amount and economic efficiency.

The hydrocarbon dispersion medium may contain at least one compound selected from the group consisting of chain aliphatic hydrocarbons having 6 to 8 carbon atoms and alicyclic hydrocarbons having 6 to 8 carbon atoms. Examples of the hydrocarbon dispersion medium include chain aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. The hydrocarbon dispersion medium may be used alone, or may be used in combination of two or more.

The hydrocarbon dispersion medium may contain at least one selected from the group consisting of n-heptane and cyclohexane from a viewpoint of industrial availability and stable quality. In addition, from the same viewpoint, as the mixture of the above-mentioned hydrocarbon dispersion medium, for example, commercially available Exxsol Heptane (manufactured by ExxonMobil: containing 75% to 85% of n-heptane and isomeric hydrocarbons) may be used.

The use amount of the hydrocarbon dispersion medium is preferably 30 to 1000 parts by mass, more preferably 40 to 500 parts by mass, and further more preferably 50 to 400 parts by mass with respect to 100 parts by mass of the monomer aqueous solution, from a viewpoint of appropriately removing the heat of polymerization and easily controlling the polymerization temperature. In a case where the use amount of the hydrocarbon dispersion medium is 30 parts by mass or more, the polymerization temperature tends to be easily controlled. In a case where the use amount of the hydrocarbon dispersion medium is 1000 parts by mass or less, the productivity of polymerization tends to be improved, which is economical.

The radical polymerization initiator is preferably water-soluble, and examples thereof include persulfates such as potassium persulfate, ammonium persulfate, and sodium persulfate; peroxides such as methyl ethyl ketone peroxide, methyl isobutyl ketone peroxide, di-t-butyl peroxide, t-butyl cumylperoxide, t-butylperoxyacetate, t-butylperoxyisobutyrate, t-butylperoxypivalate, and hydrogen peroxide; azo compounds such as 2,2'-azobis(2-amidinopropane) dihydrochloride, 2,2'-azobis [2-(N-phenylamidino) propane] dihydrochloride, 2,2'-azobis [2-(N-allylamidino) propane] dihydrochloride, 2,2'-azobis [2-(2-imidazoline-2-yl) propane] dihydrochloride, 2,2'-azobis{2-[1-(2-hydroxyethyl) -2-imidazoline-2-yl] propane} dihydrochloride, 2,2'-azobis {2-methyl-N-[1,1-bis(hydroxymethyl)-2-hydroxyethyl] propionamide}, 2,2'-azobis [2-methyl-N-(2-hydroxyethyl)-propionamide], and 4,4'-azobis (4-cyanovaleric acid). The radical polymerization initiator may be used alone, or may be used in combination of two or more. The radical polymerization initiator is preferably at least one selected from the group consisting of potassium persulfate, ammonium persulfate, sodium persulfate, 2,2'-azobis (2-amidinopropane) dihydrochloride, 2,2'-azobis [2- (2-imidazoline-2-yl) propane] dihydrochloride, and 2,2'-azobis {2-[1-(2-hydroxyethyl)-2-imidazoline-2-yl] propane} dihydrochloride.

The use amount of the radical polymerization initiator may be 0.05 to 10 mmol with respect to 1 mol of the ethylenically unsaturated monomer. In a case where the use amount of the radical polymerization initiator is 0.05 mmol or more, the polymerization reaction does not require a long time and is efficient. In a case where the use amount of the radical polymerization initiator is 10 mmol or less, the occurrence of a rapid polymerization reaction is easily inhibited.

The above-mentioned radical polymerization initiator can also be used as a redox polymerization initiator in combination with a reducing agent such as sodium sulfite, sodium hydrogen sulfite, ferrous sulfate, and L-ascorbic acid.

At the time of the polymerization reaction, the monomer aqueous solution used for the polymerization may contain a chain transfer agent. Examples of the chain transfer agent include hypophosphites, thiols, thiolic acids, secondary alcohols, and amines.

The monomer aqueous solution used for the polymerization may contain a thickener in order to control the particle diameter of the water-absorbent resin particles. Examples of the thickener include hydroxyethyl cellulose, hydroxypropyl cellulose, methyl cellulose, carboxymethyl cellulose, polyethylene glycol, polyacrylamide, polyethyleneimine, dextrin, sodium alginate, polyvinyl alcohol, polyvinylpyrrolidone, and polyethylene oxide. In a case where the stirring speed at the time of polymerization is the same, the higher the viscosity of the monomer aqueous solution, the larger the medium particle diameter of the obtained particles tends to be.

Crosslinking by self-crosslinking may occur during polymerization, but crosslinking may be performed by using an internal crosslinking agent. In a case where an internal crosslinking agent is used, the water-absorbent characteristics (water absorption rate, water retention amount, and the like) of the water-absorbent resin particles are easily controlled. The internal crosslinking agent is usually added to a reaction solution during the polymerization reaction. Examples of the internal crosslinking agent include di or tri (meth)acrylic acid esters of polyols such as ethylene glycol, propylene glycol, trimethylolpropane, glycerin, polyoxyethylene glycol, polyoxypropylene glycol, and polyglycerin; unsaturated polyesters obtained by reacting the above-mentioned polyols with unsaturated acids (such as maleic acid and fumaric acid); bis (meth)acrylamides such as N, N'-methylene bis (meth)acrylamide; di or tri (meth)acrylic acid esters obtained by reacting polyepoxide with (meth)acrylic acid; di (meth)acrylic acid carbamil esters obtained by reacting polyisocyanate (such as tolylene diisocyanate and hexamethylene diisocyanate) with hydroxyethyl (meth)acrylate; compounds having two or more polymerizable unsaturated groups such as allylated starch, allylated cellulose, diallyl phthalate, N, N', N''-triallyl isocyanurate, and divinylbenzene; polyglycidyl compounds such as (poly)ethylene glycol diglycidyl ether, (poly)propylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromhydrin, and $\alpha$-methylepichlorohydrin; and compounds having two or more reactive functional groups such as isocyanate compounds (2,4-tolylene diisocyanate and hexamethylene diisocyanate). The internal crosslinking agent may be used alone, or may be used in combination of two or more. The internal crosslinking agent may consist of a combination of compounds having two or more reactive functional groups. The internal crosslinking agent is preferably a polyglycidyl compound, more preferably a diglycidyl ether compound, and further more preferably at least one selected from the group consisting of (poly)ethylene glycol diglycidyl ether, (poly)propylene glycol diglycidyl ether, and (poly)glycerin diglycidyl ether.

The use amount of the internal crosslinking agent is preferably 30 mmol or less, more preferably 0.01 to 10 mmol, further more preferably 0.012 to 5 mmol, particularly preferably 0.015 to 1 mmol, extremely preferably 0.02 to 0.1 mmol, and extraordinarily preferably 0.025 to 0.06 mmol per 1 mol of the ethylenically unsaturated monomer, from a viewpoint of easily obtaining better permeation rate and quick-drying property, and from a viewpoint of suppressing water-soluble property by appropriately crosslinking the obtained polymer to easily obtain the sufficient water absorption amount.

It is possible to perform heating while stirring in a state of mixing an ethylenically unsaturated monomer, a radical polymerization initiator, a surfactant, a polymeric dispersant, a hydrocarbon dispersion medium, or the like (if necessary, additionally an internal crosslinking agent), and to perform reverse phase suspension polymerization in a water-in-oil system.

When performing the reverse phase suspension polymerization, a monomer aqueous solution containing an ethylenically unsaturated monomer is dispersed in a hydrocarbon dispersion medium in the presence of a surfactant (if necessary, additionally a polymeric dispersant). At this time, before the start of the polymerization reaction, the timing of adding the surfactant, the polymeric dispersant, or the like may be either before or after the addition of the monomer aqueous solution.

Among these, from a viewpoint of easily reducing the amount of the hydrocarbon dispersion medium remaining in the obtained water-absorbent resin, it is preferable to perform polymerization after dispersing the monomer aqueous solution in the hydrocarbon dispersion medium in which the polymeric dispersant is dispersed and then further dispersing the surfactant.

Reverse phase suspension polymerization can be performed in one stage, or in multiple stages of two or more stages. Reverse phase suspension polymerization is preferably performed in two to three stages from a viewpoint of increasing productivity.

In a case where reverse phase suspension polymerization is performed in multiple stages of two or more stages, a first stage reverse phase suspension polymerization is performed, an ethylenically unsaturated monomer is added to the reaction mixture obtained in the first polymerization reaction and mixed therewith, and second and subsequent stages of reverse phase suspension polymerization may be performed in the same method as the first stage. In the reverse phase suspension polymerization in each stage of the second and subsequent stages, in addition to the ethylenically unsaturated monomer, the above-mentioned radical polymerization initiator and/or internal crosslinking agent is preferably added in a range of a molar ratio of each component with respect to the above-mentioned ethylenically unsaturated monomer, based on an amount of the ethylenically unsaturated monomer added at the time of the second and subsequent stages of reverse phase suspension polymerization, to perform reverse phase suspension polymerization. In the reverse phase suspension polymerization in each stage of second and subsequent stages, an internal crosslinking agent may be used if necessary. In a case of using the internal crosslinking agent, the internal crosslinking agent is preferably added within a range of the molar ratio of each component with respect to the above-mentioned ethylenically unsaturated monomer based on the amount of the ethylenically unsaturated monomer provided in each stage, to perform reverse phase suspension polymerization.

The temperature of the polymerization reaction varies depending on the used radical polymerization initiator, and the temperature is preferably 20°C to 150°C, and more preferably 40°C to 120°C, from a viewpoint of rapidly proceeding the polymerization and shortening the polymerization time to enhance economic efficiency, and easily removing polymerization heat and smoothly performing reaction. The reaction time is usually 0.5 to 4 hours. The completion of the polymerization reaction can be confirmed by stopping the temperature rise in the reaction system. Thus, the polymer of the ethylenically unsaturated monomer is usually obtained in a state of a hydrogel.

After the polymerization, a post-polymerization crosslinking agent may be added to the obtained hydrogel-like polymer and heated to perform crosslinking. By performing post-polymerization crosslinking, a degree of crosslinking of the hydrogel-like polymer can be increased, and the water-absorbent characteristics (water absorption rate, water retention amount, and the like) can be further improved.

Examples of the post-polymerization crosslinking agent include polyols such as ethylene glycol, propylene glycol, 1,4-butanediol, trimethylolpropane, glycerin, polyoxyethylene glycol, polyoxypropylene glycol, and polyglycerin; compounds having two or more epoxy groups such as (poly)ethylene glycol diglycidyl ether, (poly)propylene glycol diglycidyl ether, and (poly)glycerin diglycidyl ether; haloepoxy compounds such as epichlorohydrin, epibromhydrin, and $\alpha$-methylepichlorohydrin; compounds having two or more isocyanate groups such as 2,4-tolylene diisocyanate and hexamethylene diisocyanate; oxazoline compounds such as 1,2-ethylenebisoxazoline; carbonate compounds such as ethylene carbonate; and hydroxyalkylamide compounds such as bis [N, N-di ($\beta$-hydroxyethyl)] adipamide. Among these, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are preferable. The crosslinking agent may be used alone, or may be used in combination of two or more.

The amount of the post-polymerization crosslinking agent is preferably 30 mmol or less, more preferably 10 mmol or less, further more preferably 0.01 to 5 mmol, particularly preferably 0.012 to 1 mmol, extremely preferably 0.015 to 0.1 mmol, and extraordinarily preferably 0.02 to 0.05 mmol per 1 mol of the ethylenically unsaturated monomer, from a viewpoint of easily obtaining suitable water-absorbent characteristics (water absorption rate, water retention amount, and the like) by appropriately crosslinking the obtained hydrogel-like polymer.

The timing of adding the post-polymerization crosslinking agent may be after the polymerization of the ethylenically unsaturated monomer used for the polymerization, and in the case of multiple-stage polymerization, it is preferable to add after the multiple-stage polymerization. Considering fluctuation in water due to heat generation at the time of polymerization and after polymerization, retention due to process delay, opening of the system at the time of addition of the crosslinking agent, addition of water due to the addition of the crosslinking agent, or the like, the post-polymerization crosslinking agent is preferably added in a region of [water content (immediately after polymerization) $\pm$ 3% by mass] from a viewpoint of water content (to be described later).

Subsequently, the polymer particles (for example, polymer particles having a structural unit derived from an ethylenically unsaturated monomer) are obtained by drying in order to remove water from the obtained hydrogel-like polymer. Examples of a drying method include (a) a method of removing water by performing azeotropic distillation by heating from outside in a state where a hydrogel-like polymer is dispersed in a hydrocarbon dispersion medium, and refluxing the hydrocarbon dispersion medium, (b) a method of taking out a hydrogel-like polymer by decantation and drying under reduced pressure, and (c) a method of filtering the hydrogel-like polymer with a filter and drying under reduced pressure. Among these, it is preferable to use the method (a) due to the simplicity in the production process.

It is possible to adjust the particle diameter of water-absorbent resin particles by adjusting a rotation speed of a stirrer during the polymerization reaction, or by adding a flocculant into the system after the polymerization reaction or in the initial stage of drying. By adding a flocculant, it is possible to increase the particle diameter of the obtained water-absorbent resin particles. As the flocculant, an inorganic flocculant can be used. Examples of the inorganic flocculant (for example, powdered inorganic flocculant) include silica, zeolite, bentonite, aluminum oxide, talc, titanium dioxide, kaolin, clay, and hydrotalcite. From a viewpoint of better flocculation effect, the flocculant is preferably at least one selected from the group consisting of silica, aluminum oxide, talc, and kaolin.

In the reverse phase suspension polymerization, a method of adding the flocculant is preferably a method of preliminarily dispersing a flocculant in a hydrocarbon dispersion medium or water of the same type as that used in the polymerization, and then mixing into a hydrocarbon dispersion medium containing a hydrogel-like polymer under stirring.

The addition amount of the flocculant is preferably 0.001 to 1 part by mass, more preferably 0.005 to 0.5 part by mass, and further more preferably 0.01 to 0.2 parts by mass with respect to 100 parts by mass of the ethylenically unsaturated monomer used for the polymerization. In a case where the addition amount of the flocculant is within the above-mentioned range, water-absorbent resin particles having a target particle size distribution can be easily obtained.

In the production of the water-absorbent resin particles, it is preferable to perform surface crosslinking of a surface portion (surface and in the vicinity of surface) of a hydrogel-like polymer using a surface crosslinking agent in a drying step (water removing step) or any subsequent steps. By performing surface crosslinking, the water-absorbent characteristics (water absorption rate, water retention amount, and the like) of the water-absorbent resin particles is easily controlled. The surface crosslinking is preferably performed at the timing when the hydrogel-like polymer has a specific water content. The timing of surface crosslinking is preferably when the water content of the hydrogel-like polymer is 5% to 50% by mass, more preferably when the water content of the hydrogel-like polymer is 10% to 40% by mass, and further more preferably when the water content of the hydrogel-like polymer is 15% to 35% by mass. The water content (mass%) of the hydrogel-like polymer is calculated by the following formula.

$$\text{Water content} = [\text{Ww}/(\text{Ww} + \text{Ws})] \times 100$$

Ww: water amount of a hydrogel-like polymer obtained by adding water amount used if necessary when mixing a flocculant, a surface crosslinking agent, or the like to an amount obtained by subtracting water amount discharged to the outside of the system in the drying step, from water amount contained in a monomer aqueous solution before polymerization in the entire polymerization step.
Ws: Solid content calculated from the charged amount of materials such as ethylenically unsaturated monomer, crosslinking agent, and initiator that constitute a hydrogel-like polymer.

Examples of the surface crosslinking agent include compounds having two or more reactive functional groups. Examples of the surface crosslinking agent include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylpropane triglycidyl ether (poly)propylene glycol polyglycidyl ether, and (poly)glycerol polyglycidyl ether; haloepoxy compounds such as epichlorohydrin, epibromhydrin, and $\alpha$-methylepichlorohydrin; isocyanate compounds such as 2,4-tolylene diisocyanate and hexamethylenediisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; and hydroxyalkylamide compounds such as bis [N, N-di (β-hydroxyethyl)]adipamide. The surface crosslinking agent may be used alone, or may be used in combination of two or more. The surface crosslinking agent is preferably a polyglycidyl compound, and more preferably at least one selected from the group consisting of (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether.

The use amount of the surface crosslinking agent is preferably 0.01 to 20 mmol, more preferably 0.05 to 10 mmol, further more preferably 0.1 to 5 mmol, particularly preferably 0.15 to 1 mmol, and extremely preferably 0.2 to 0.5 mmol per 1 mol of the ethylenically unsaturated monomer used for polymerization, from a viewpoint of easily obtaining suitable water-absorbent characteristics (water absorption rate, water retention amount, and the like).

After surface crosslinking, it is possible to obtain polymer particles which are surface-cross-linked dried products by distilling water and a hydrocarbon dispersion medium, heating and drying under reduced pressure, or the like, with a known method.

As described above, the polymer particles contained in the water-absorbent resin particles can be obtained by using an internal crosslinking agent used at the time of polymerizing the monomer, and can be obtained by using an internal crosslinking agent, and an external crosslinking agent (a post-polymerization crosslinking agent used after the polymerization of the monomer, and a surface crosslinking agent used in the drying step after polymerization of a monomer or subsequent steps) used after polymerization of the monomer. The ratio of the use amount of the external crosslinking agent with respect to the internal crosslinking agent (external crosslinking agent/internal crosslinking agent) is preferably 5 to 100, more preferably 6 to 80, further more preferably 8 to 60, particularly preferably 10 to 40, and extremely preferably 10 to 30, from a viewpoint of easily obtaining suitable water-absorbent characteristics (water absorption rate, water retention amount, and the like). The water-absorbent resin particles may contain polymer particles which are reaction products using an internal crosslinking agent, and may contain polymer particles which are reaction products using an internal crosslinking agent and an external crosslinking agent. The ratio of the use amount of the external crosslinking agent with respect to the internal crosslinking agent in the polymer particles is preferably in the above range.

In addition to the polymer particles, the water-absorbent resin particles of the present embodiment can further contain additional components such as a gel stabilizer, a metal chelating agent (ethylenediaminetetraacetic acid and a salt thereof, diethylenetriamine pentaacetate and a salt thereof, and the like, for example, diethylenetriamine pentasodium pentaacetate), and a flowability improver (lubricant). Additional components can be disposed inside the polymer particles, on the surface of the polymer particles, or both thereof.

The water-absorbent resin particles may contain a plurality of inorganic particles disposed on the surface of the polymer particles. For example, by mixing the polymer particles and the inorganic particles, it is possible to dispose the inorganic particles on the surface of the polymer particles. The inorganic particles may be silica particles such as amorphous silica.

In a case where the water-absorbent resin particles include inorganic particles disposed on the surface of the polymer particles, the content of the inorganic particles may be in the following range based on the total mass of the polymer particles. The content of the inorganic particles may be 0.05% by mass or more, 0.1% by mass or more, 0.15% by mass or more, or 0.2% by mass or more. The content of the inorganic particles may be 5.0% by mass or less, 3.0% by mass or less, 1.0% by mass or less, or 0.5% by mass or less.

The inorganic particles here usually have a minute size as compared with the size of the polymer particles. For example, the average particle diameter of the inorganic particles may be 0.1 to 50 $\mu$m, 0.5 to 30 $\mu$m, or 1 to 20 $\mu$m. The average particle diameter can be measured by a pore electric resistance method or a laser diffraction/scattering method depending on the characteristics of the particles.

The absorber of the present embodiment contains the water-absorbent resin particles of the present embodiment. The absorber of the present embodiment may contain a fibrous substance, for example, is a mixture containing water-absorbent resin particles and the fibrous substance. For example, the structure of the absorber may be a structure in which the water-absorbent resin particles and the fibrous substance are uniformly mixed, may be a structure in which the water-absorbent resin particles are sandwiched between the fibrous substances formed in the form of a sheet or a layer, or may be other structures.

Examples of the fibrous substance include finely pulverized wood pulp; cotton; cotton linter; rayon; cellulosic fibers such as cellulose acetate; synthetic fibers such as polyamide, polyester and polyolefin; and a mixture of these fibers. The fibrous substance may be used alone, or may be used in combination of two or more. As the fibrous substance, hydrophilic fibers can be used.

In order to enhance the morphological retention before and during use of the absorber, the fibers may be adhered to each other by adding an adhesive binder to the fibrous substance. Examples of the adhesive binder include thermal bonding synthetic fibers, hot melt adhesives, and adhesive emulsions. The adhesive binder may be used alone, or may be used in combination of two or more.

Examples of the thermal bonding synthetic fiber include a total fusion type binder such as polyethylene, polypropylene, and an ethylene-propylene copolymer; and a non-total fusion type binder made of a side-by-side or core-sheath structure of polypropylene and polyethylene. In the above-mentioned non-total fusion type binder, only the polyethylene portion can be thermal-bonded.

Examples of the hot melt adhesive include a mixture of a base polymer such as ethylene-vinyl acetate copolymer, styrene-isoprene-styrene block copolymer, styrene-butadiene-styrene block copolymer, styrene-ethylene-butylene-styrene block copolymer, styrene-ethylene-propylene-styrene block copolymer, and amorphous polypropylene with a tackifier, a plasticizer, an antioxidant, or the like.

Examples of the adhesive emulsion include a polymerization product of at least one monomer selected from the group consisting of methyl methacrylate, styrene, acrylonitrile, 2-ethylhexyl acrylate, butyl acrylate, butadiene, ethylene, and vinyl acetate.

The absorber of the present embodiment may contain an inorganic powder (for example, amorphous silica), a deodorant, an antibacterial agent, a dye, a pigment, a fragrance, a sticking agent, or the like. In a case where the water-absorbent resin particles contain inorganic particles, the absorber may contain an inorganic powder in addition to the inorganic particles of the water-absorbent resin particles.

The shape of the absorber of the present embodiment may be a sheet shape, for example. The thickness of the absorber (for example, thickness of the sheet shaped absorber) may be 0.1 to 20 mm or 0.3 to 15 mm.

The content of the water-absorbent resin particles in the absorber may be 2% to 100% by mass, 10% to 80% by mass, or 20% to 60% by mass with respect to the total of the water-absorbent resin particles and the fibrous substance, from a viewpoint of easily obtaining sufficient water absorption performance.

The content of the water-absorbent resin particles in the absorber is preferably 100 to 1000 g, more preferably 150 to 800 g, and further more preferably 200 to 700 g per 1 $m^2$ of the absorber from a viewpoint of easily obtaining sufficient water absorption performance. The content of the fibrous substance in the absorber is preferably 50 to 800 g, more preferably 100 to 600 g, and further more preferably 150 to 500 g per 1 $m^2$ of the absorber from a viewpoint of easily obtaining sufficient water absorption performance.

The absorbent article of the present embodiment includes an absorber of the present embodiment. Examples of the absorbent article of the present embodiment include a core wrap that retains an absorber and prevents falloff or flow of a constituent member of the absorber; a liquid permeable sheet disposed on the outermost part at the side where the liquid to be absorbed enters; and a liquid impermeable sheet disposed on the outermost part at the opposite side to the side where the liquid to be absorbed enters. Examples of the absorbent article include diapers (for example, paper diapers), toilet training pants, incontinence pads, sanitary materials (sanitary napkins, tampons, and the like), sweat pads, pet sheets, portal toilet members, and animal excrement treatment materials.

Fig. 1 is a cross-sectional view showing an example of an absorbent article. An absorbent article 100 shown in Fig. 1 includes an absorber 10, core wraps 20a and 20b, a liquid permeable sheet 30, and a liquid impermeable sheet 40. In the absorbent article 100, the liquid impermeable sheet 40, the core wrap 20b, the absorber 10, the core wrap 20a, and the liquid permeable sheet 30 are laminated in this order. In Fig. 1, there is a portion shown so that there is a gap between the members, but the members may be in close contact with each other without the gap.

The absorber 10 has a water-absorbent resin particle 10a of the present embodiment and a fiber layer 10b containing a fibrous substance. The water-absorbent resin particles 10a are dispersed in the fiber layer 10b.

The core wrap 20a is disposed on one surface side of the absorber 10 (upper side of the absorber 10 in Fig. 1) in a state of being in contact with the absorber 10. The core wrap 20b is disposed on the other surface side of the absorber 10 (lower side of the absorber 10 in Fig. 1) in a state of being in contact with the absorber 10. The absorber 10 is disposed between the core wrap 20a and the core wrap 20b. Examples of the core wraps 20a and 20b include tissues, non-woven fabrics, woven fabrics, synthetic resin films having liquid permeation holes, and net-like sheets having a mesh. The core wrap 20a and the core wrap 20b have a main surface having the same size as that of the absorber 10, for example.

The liquid permeable sheet 30 is disposed on the outermost part at the side where the liquid to be absorbed enters. The liquid permeable sheet 30 is disposed on the core wrap 20a in a state of being in contact with the core wrap 20a. Examples of the liquid permeable sheet 30 include a non-woven fabric made of a synthetic resin such as polyethylene, polypropylene, polyester, and polyamide, and a porous sheet. The liquid impermeable sheet 40 is disposed on the outermost part at the opposite side to the liquid permeable sheet 30 in the absorbent article 100. The liquid impermeable sheet 40 is disposed on a lower side of the core wrap 20b in a state of being in contact with the core wrap 20b. Examples of the liquid impermeable sheet 40 include a sheet made of a synthetic resin such as polyethylene, polypropylene, and polyvinyl chloride, and a sheet made of a composite material of these synthetic resins and a non-woven fabric. The liquid permeable sheet 30 and the liquid impermeable sheet 40 have a main surface wider than the main surface of the absorber 10, and outer edges of the liquid permeable sheet 30 and the liquid impermeable sheet 40 are present around the absorber 10 and the core wraps 20a and 20b.

The magnitude relationship between the absorber 10, the core wraps 20a and 20b, the liquid permeable sheet 30, and the liquid impermeable sheet 40 is not particularly limited, and is appropriately adjusted according to the use of the absorbent article or the like. In addition, the method of retaining the shape of the absorber 10 using the core wraps 20a and 20b is not particularly limited, and as shown in Fig. 1, the absorber may be wrapped by a plurality of core wraps, and the absorber is wrapped by one core wrap.

The absorber may be adhered to a top sheet. In a case where the absorber is sandwiched or covered by the core wrap, it is preferable that at least the core wrap and the top sheet are adhered to each other, and it is more preferable that the core wrap and the top sheet are adhered to each other and the core wrap and the absorber are adhered to each other. Examples of a method of adhering the absorber include a method of adhering by applying a hot melt adhesive to the top sheet at predetermined intervals in a striped shape, a spiral shape, or the like in a width direction; and a method of adhering using a water-soluble binder such as starch, carboxymethyl cellulose, polyvinyl alcohol, polyvinylpyrrolidone, and other water-soluble polymers. In addition, in a case where the absorber contains thermal bonding synthetic fibers, a method of adhering by thermal bonding of the thermal bonding synthetic fibers may be adopted.

According to the present embodiment, it is possible to provide a liquid absorbing method using the water-absorbent resin particles, the absorber or the absorbent article of the present embodiment. The liquid absorbing method of the present embodiment includes a step of bringing the liquid to be absorbed into contact with the water-absorbent resin particles, the absorber or the absorbent article of the present embodiment.

According to the present embodiment, it is possible to provide a method for improving the permeation rate of an absorbent article, which is a method for improving the permeation rate using the water-absorbent resin particles, the absorber or the absorbent article of the present embodiment. According to the present embodiment, it is possible to provide a method for producing water-absorbent resin particles, including a selection step of selecting water-absorbent resin particles based on a water absorption rate based on a low-speed flow Vortex method (Vortex method at 300 rpm). In the selection step, for example, the water-absorbent resin particles are selected based on whether or not the water absorption rate based on the low-speed flow Vortex method is 10 to 50 seconds.

**Examples**

Hereinafter, contents of the present invention will be described in further detail using examples and comparative examples, but the present invention is not limited to the following examples.

<Preparation of water-absorbent resin particles>

(Example 1)

A round-bottomed cylindrical separable flask with the inner diameter of 11 cm and the internal volume of 2 L equipped with a reflux cooling device, a dropping funnel, a nitrogen gas introduction tube, and a stirrer (a stirrer blade having two stages of four inclined paddle blades with the blade diameter of 5 cm) was prepared. Into this flask, 293 g of n-heptane was added as a hydrocarbon dispersion medium and 0.736 g of a maleic anhydride-modified ethylene/propylene copolymer (manufactured by Mitsui Chemicals, Inc., High Wax 1105A) was added as a polymeric dispersant to obtain a mixture. The dispersant was dissolved by raising the temperature to 80°C while stirring the mixture, and then the mixture was cooled to 50°C.

Subsequently, 92.0 g of 80.5% by mass aqueous acrylic acid solution (acrylic acid: 1.03 mol) was added into a beaker having the internal volume of 300 mL as a water-soluble ethylenically unsaturated monomer. Subsequently, while cooling from the outside, 147.7 g of 20.9% by mass sodium hydroxide aqueous solution was added dropwise into the beaker to perform 75 mol% of neutralization. Thereafter, 0.092 g of hydroxyethyl cellulose (manufactured by Sumitomo Seika Chemicals Co., Ltd., HEC AW-15F) as a thickener, 0.0736 g (0.272 mmol) of potassium persulfate as a water-soluble radical polymerization initiator, and 0.010 g (0.057 mmol) of ethylene glycol diglycidyl ether as an internal crosslinking agent were added and then dissolved therein to prepare a first stage aqueous solution.

Then, the first stage aqueous solution was added into the above-mentioned separable flask while stirring at the rotation speed of 550 rpm of the stirrer, and then stirring was performed for 10 minutes. Thereafter, a surfactant solution obtained by heat-dissolving 0.736 g of sucrose stearic acid ester (surfactant, manufactured by Mitsubishi-Chemical Foods Corporation, Ryoto Sugar Ester S-370, HLB value: 3) in 6.62 g of n-heptane was added into the separable flask. Then, the inside of the system was sufficiently replaced with nitrogen while stirring at the stirring speed of 550 rpm of the stirrer. Thereafter, the flask was immersed in a water bath at 70°C to raise the temperature, and polymerization was performed for 60 minutes to obtain a first stage polymerization slurry solution.

Subsequently, 128.8 g of 80.5% by mass aqueous acrylic acid solution (acrylic acid: 1.43 mol) was added into another beaker having the internal volume of 500 mL as a water-soluble ethylenically unsaturated monomer. Subsequently, while cooling from the outside, 159.0 g of 27% by mass sodium hydroxide aqueous solution was added dropwise into the beaker to perform 75 mol% of neutralization. Thereafter, 0.090 g (0.334 mmol) of potassium persulfate was added as a water-soluble radical polymerization initiator and then dissolved therein to prepare a second stage aqueous solution.

Subsequently, while stirring at the rotation speed of 1000 rpm of the stirrer, the inside of the above-mentioned separable flask was cooled to 25°C, and then the total amount of the above-mentioned second stage aqueous solution was added to the above-mentioned first stage polymerization slurry solution. Subsequently, after replacing the inside of the system with nitrogen for 30 minutes, the flask was immersed in a water bath at 70°C again to raise the temperature, and the polymerization reaction was performed for 60 minutes. Thereafter, 0.580 g of 2% by mass ethylene glycol diglycidyl ether aqueous solution (ethylene glycol diglycidyl ether: 0.067 mmol) was added as a post-polymerization crosslinking agent to obtain a second stage hydrogel-like polymer.

To the above-mentioned second stage hydrogel-like polymer, 0.265 g of 45% by mass diethylenetriamine pentasodium pentaacetate aqueous solution was added under stirring. Thereafter, the flask was immersed in an oil bath set at 125°C, and 241.9 g of water was extracted to the outside of the system while refluxing n-heptane by azeotropic distillation of n-heptane and water. Then, 4.42 g of 2% by mass ethylene glycol diglycidyl ether aqueous solution (ethylene glycol diglycidyl ether: 0.507 mmol) was added into the flask as a surface crosslinking agent, and then the mixture was held at 83°C for 2 hours.

Thereafter, n-heptane was evaporated at 125°C and dried to obtain polymer particles (dried product). After passing these polymer particles through a sieve having the opening of 850 μm, 0.5% by mass of amorphous silica (Tokusil NP-S manufactured by Oriental Silicas Corporation) was mixed with the polymer particles based on the total mass of the polymer particles to obtain 229.2 g of water-absorbent resin particles containing amorphous silica. The medium particle diameter of the water-absorbent resin particles was 377 μm. In Example 1, the ratio of the use amount of the external crosslinking agent with respect to the use amount of the internal crosslinking agent was 10.1 in terms of molar ratio.

(Example 2)

231.0 g of water-absorbent resin particles were obtained in the same manner as that in Example 1, except that, in the hydrogel-like polymer after the second stage polymerization, 247.9 g of water was extracted to the outside of the system by azeotropic distillation. The medium particle diameter of the water-absorbent resin particles was 355 μm.

(Example 3)

229.6 g of water-absorbent resin particles were obtained in the same manner as that in Example 1, except that, in the preparation of the first stage aqueous solution, 0.092 g (0.339 mmol) of 2,2'-azobis (2-amidinopropane) dihydrochloride and 0.018 g (0.068 mmol) of potassium persulfate were used as water-soluble radical polymerization initiators, and 0.0045 g (0.026 mmol) of ethylene glycol diglycidyl ether was used as the internal crosslinking agent; in the preparation of the second stage aqueous solution, 0.129 g (0.475 mmol) of 2,2'-azobis (2-amidinopropane) dihydrochloride and 0.026 g (0.095 mmol) of potassium persulfate were used as water-soluble radical polymerization initiators; in the hydrogel-like polymer after the second polymerization, 223.7 g of water was extracted to the outside of the system by azeotropic distillation; and 0.2% by mass amorphous silica with respect to the mass of the polymer particles was mixed with polymer particles. The medium particle diameter of the water-absorbent resin particles was 346 μm. In Example 3, the ratio of the use amount of the external crosslinking agent with respect to the use amount of the internal crosslinking agent was 22.1 in terms of molar ratio.

(Example 4)

230.1 g of water-absorbent resin particles were obtained in the same manner as that in Example 1, except that, in the hydrogel-like polymer after the second stage polymerization, 256.1 g of water was extracted to the outside of the system by azeotropic distillation. The medium particle diameter of the water-absorbent resin particles was 364 μm.

(Example 5)

231.1 g of water-absorbent resin particles were obtained in the same manner as that in Example 1, except that, in the hydrogel-like polymer after the second stage polymerization, 264.3 g of water was extracted to the outside of the system by azeotropic distillation. The medium particle diameter of the water-absorbent resin particles was 361 μm.

(Example 6)

230.5 g of water-absorbent resin particles were obtained in the same manner as that in Example 1, except that, in the preparation of the first stage aqueous solution, 0.092 g (0.339 mmol) of 2,2'-azobis (2-amidinopropane) dihydrochloride and 0.018 g (0.068 mmol) of potassium persulfate were used as water-soluble radical polymerization initiators, and 0.0045 g (0.026 mmol) of ethylene glycol diglycidyl ether was used as an internal crosslinking agent; in the preparation of the second stage aqueous solution, 0.129 g (0.475 mmol) of 2,2'-azobis (2-amidinopropane) dihydrochloride and 0.026 g (0.095 mmol) of potassium persulfate were used as water-soluble radical polymerization initiators, and 0.0117 g (0.067 mmol) of ethylene glycol diglycidyl ether was used as an internal crosslinking agent; in the preparation of the hydrogel-like polymer, after performing polymerization reaction for 60 minutes, 0.265 g of 45% by mass diethylenetriamine pentasodium pentaacetate aqueous solution was added without adding a post-polymerization crosslinking agent; in the hydrogel-like polymer after the second polymerization, 217.8 g of water was extracted to the outside of the system by azeotropic distillation; and in the preparation of polymer particles, instead of evaporating n-heptane at 125°C, immediately after surface crosslinking reaction, a highly water-absorbent resin hydrate obtained by filtering an n-heptane phase from the reaction solution with a sieve having the opening of 38 μm was heated and dried under reduced pressure of 0.006 MPa using a decompression drier set at 90°C. The medium particle diameter of the water-absorbent resin particles was 367 μm. In Example 6, the ratio of the use amount of the external crosslinking agent with respect to the use amount of the internal crosslinking agent was 5.5 in terms of molar ratio.

(Example 7)

230.8 g of water-absorbent resin particles were obtained in the same manner as that in Example 1, except that the rotation speed of the stirrer was changed to 500 rpm in the preparation of the first stage polymerization slurry solution, 256.1 g of water was extracted to the outside of the system by azeotropic distillation in the hydrogel-like polymer after the second stage polymerization, and 0.1% by mass amorphous silica with respect to the mass of the polymer particles was mixed with the polymer particles. The medium particle diameter of the water-absorbent resin particles was 349 μm.

(Comparative Example 1)

230.8 g of water-absorbent resin particles were obtained in the same manner as that in Example 1, except that, in the preparation of the second stage aqueous solution, 0.0117 g (0.067 mmol) of ethylene glycol diglycidyl ether was used as an internal crosslinking agent in addition to the water-soluble radical polymerization initiator; in the preparation of the hydrogel-like polymer, after performing polymerization reaction for 60 minutes, 0.265 g of 45 mass% diethylenetriamine pentasodium pentaacetate aqueous solution was added without adding the post-polymerization crosslinking agent; in the hydrogel-like polymer after the second stage polymerization, 278.9 g of water was extracted to the outside of the system by azeotropic distillation; and 0.2% by mass amorphous silica with respect to the mass of the polymer particles was mixed with the polymer particles. In Comparative Example 1, the ratio of the use amount of the external crosslinking agent with respect to the use amount of the internal crosslinking agent was 4.1 in terms of molar ratio.

(Comparative Example 2)

229.6 g of water-absorbent resin particles were obtained in the same manner as that in Example 1, except that, in the preparation of the first-stage aqueous solution, 0.092 g (0.339 mmol) of 2,2'-azobis (2-amidinopropane) dihydrochloride and 0.018 g (0.068 mmol) of potassium persulfate were used as water-soluble radical polymerization initiators, and 0.0045 g (0.026 mmol) of ethylene glycol diglycidyl ether was used as the internal crosslinking agent; in the preparation of the second stage aqueous solution, 0.129 g (0.475 mmol) of 2,2'-azobis (2-amidinopropane) dihydrochloride and 0.026 g (0.095 mmol) of potassium persulfate were used as water-soluble radical polymerization initiators, and 0.0117 g (0.067 mmol) of ethylene glycol diglycidyl ether was used as an internal crosslinking agent; in the preparation of the hydrogel-like polymer, after performing polymerization reaction for 60 minutes, 0.265 g of 45% by mass diethylenetriamine pentasodium pentaacetate aqueous solution was added without adding the post-polymerization crosslinking agent; in the hydrogel-like polymer after the second stage polymerization, 233.5 g of water was extracted to the outside of the system by azeotropic distillation; and 0.2% by mass amorphous silica with respect to the mass of the polymer particles was mixed with polymer particles. In Comparative Example 2, the ratio of the use amount of the external crosslinking agent with respect to the use amount of the internal crosslinking agent was 5.5 in terms of molar ratio.

(Comparative Example 3)

229.6 g of water-absorbent resin particles were obtained in the same manner as that in Comparative Example 2, except that, in the hydrogel-like polymer after the second stage polymerization, 245.1 g of water was extracted to the outside of the system by azeotropic distillation.

(Comparative Example 4)

A round-bottomed cylindrical separable flask having 4 side wall baffles (baffle width: 7 mm), with the inner diameter of 11 cm and the internal volume of 2 L, equipped with a reflux cooling device, a dropping funnel, a nitrogen gas introduction tube, and a stirrer (a stirrer blade having two stages of four inclined paddle blades with the blade diameter of 5 cm (those surface-treated with fluororesin)) was prepared. Into this flask, 451.4 g of n-heptane as a hydrocarbon dispersion medium was added, and 1.288 g of sorbitan monolaurate (Nonion LP-20R, HLB value: 8.6, manufactured by NOF CORPORATION) was added as a surfactant to obtain a mixture. The sorbitan monolaurate was dissolved in n-heptane by raising the temperature of this mixture to 50°C while stirring at the rotation speed of 300 rpm of the stirrer, and then the mixture was cooled to 40°C.

Subsequently, 92.0 g of 80.5% by mass aqueous acrylic acid solution (acrylic acid: 1.03 mol) was put into a triangular flask having the internal volume of 500 mL. Subsequently, while cooling with ice from the outside, 147.7 g of 20.9% by mass sodium hydroxide aqueous solution was added dropwise to neutralize the acrylic acid to obtain an acrylic acid partial neutralization aqueous solution. Subsequently, 0.1012 g (0.374 mmol) of potassium persulfate as a water-soluble radical polymerization initiator was added to the acrylic acid partial neutralization aqueous solution, and then dissolved therein to prepare a monomer aqueous solution.

After adding the above-mentioned monomer aqueous solution into the above-mentioned separable flask, the inside of the system was sufficiently replaced with nitrogen. Thereafter, while stirring at the rotation speed of 700 rpm of the stirrer, the flask was immersed in a water bath at 70°C and held for 60 minutes to complete the polymerization to obtain a hydrogel-like polymer.

Thereafter, while stirring at the stirring speed of 1000 rpm of the stirrer, a dispersion obtained by previously dispersing 0.092 g of amorphous silica (Oriental Silicas Corporation, Tokusil NP-S) as a powdered inorganic flocculant to 100 g of n-heptane was added to a polymer solution containing the produced hydrogel-like polymer, n-heptane, and a surfactant, and then mixing was performed for 10 minutes. Thereafter, the flask containing the reaction solution was immersed in an oil bath at 125°C, and 129.0 g of water was extracted to the outside of the system while refluxing n-heptane by azeotropic distillation of n-heptane and water. Thereafter, 4.14 g of 2% by mass ethylene glycol diglycidyl ether aqueous solution (ethylene glycol diglycidyl ether: 0.475 mmol) was added as a surface crosslinking agent, and then the internal temperature was held at 83 $\pm$ 2°C for 2 hours.

Thereafter, water and n-heptane were evaporated at 120°C and dried until almost no evaporated product from the system was distilled off to obtain a dried product. This dried product was passed through a sieve having the opening of 850 $\mu$m to obtain 90.1 g of water-absorbent resin particles.

<Measurement of medium particle diameter>

The above-mentioned medium particle diameter of the water-absorbent resin particles was measured by the following procedure. Specifically, JIS standard sieves were combined in the following order from the top: a sieve having the opening of 600 $\mu$m, a sieve having the opening of 500 $\mu$m, a sieve having the opening of 425 $\mu$m, a sieve having the opening of 300 $\mu$m, a sieve having the opening of 250 $\mu$m, a sieve having the opening of 180 $\mu$m, a sieve having the opening of 150 $\mu$m, and a tray. 50 g of the water-absorbent resin particles were put in the topmost sieve among the combined sieves, and classification was performed by shaking for 10 minutes using a Ro-tap shaker. After classification, the mass of the particles remaining on each sieve was calculated as a mass percentage with respect to the total amount, and the particle size distribution was obtained. The relationship between the opening of the sieve and the integrated value of the mass percentage of the particles remaining on the sieve was plotted on logarithmic probability paper by integrating in the order from the one having the largest particle diameter on the sieve with respect to this particle size distribution. By connecting the plots on the probability paper with a straight line, the particle diameter corresponding to the cumulative mass percentage of 50% by mass was obtained as the medium particle diameter.

<Water absorption rate of water-absorbent resin particles>

The water absorption rate of physiological saline of the water-absorbent resin particles was measured by the following procedure based on the Vortex method. First, 50 $\pm$ 0.1 g of physiological saline adjusted to the temperature of 25 $\pm$ 0.2°C in a constant temperature water tank was weighed in a beaker having the internal volume of 100 mL. Subsequently, a vortex was generated by stirring at the rotation speed of 300 rpm (low-speed flow Vortex method) or 600 rpm (Vortex method in the related art) using a magnetic stirrer bar (8 mm$\varphi$ $\times$ 30 mm, without ring). 2.0 $\pm$ 0.002 g of the water-absorbent resin particles were added to physiological saline at one time. The time [seconds] from after the addition of the water-absorbent resin particles until the vortex on the liquid surface converged was measured, and this time was obtained as the water absorption rate of the water-absorbent resin particles. The results are shown in Table 1.

<Water retention amount of water-absorbent resin particles>

The water retention amount (room temperature, 25 $\pm$ 2°C) of physiological saline of the water-absorbent resin particles was measured by the following procedure. First, a cotton bag (Membroroad No. 60, width 100 mm $\times$ length 200 mm) containing 2.0 g of the weighted water-absorbent resin particles was placed in a beaker having the internal volume of 500 mL. After pouring 500 g of physiological saline into a cotton bag containing the water-absorbent resin particles at one time so that lumps cannot be formed, an upper portion of the cotton bag was tied with a rubber band and the cotton bag was left for 30 minutes to swell the water-absorbent resin particles. The cotton bag after 30 minutes was dehydrated for 1 minute using a dehydrator (product number: H-122, manufactured by KOKUSAN Co., Ltd.) set to have the centrifugal force of 167 G, and then the mass Wa [g] of the cotton bag containing swollen gel after dehydration was measured. The same operation was performed without adding the water-absorbent resin particles, the empty mass Wb [g] at the time when the cotton bag was wet was measured, and the water retention amount of physiological saline of the water-absorbent resin particles was calculated from the formula below. The results are shown in Table 1.

$$\text{Water retention amount [g/g]} = (Wa - Wb)/2.0$$

<Preparation of absorbent article>

Using an air flow type mixer (Padformer, manufactured by O-tec Co., Ltd.), 13.3 g of the water-absorbent resin particles and 12.6 g of pulverized pulp were uniformly mixed by air papermaking to prepare a sheet-shaped absorber having the size of 40 cm × 12 cm. Subsequently, in a state where the upper and lower sides of the absorber were sandwiched between two sheets of tissue paper having the same size as that of the sheet-shaped absorber and having the basis weight of 16 g/m², the load of 424 kPa was applied to the entire body for 30 seconds and pressed to form a laminate. In addition, an air-through type porous liquid permeable sheet made of polyethylene-polypropylene having the same size as that of the absorber and having the basis weight of 22 g/m² was disposed on the upper surface of the laminate to prepare an absorbent article.

<Measurement of permeation time>

An absorbent article was disposed on a horizontal table in a room at the temperature of 25 ± 2°C. Subsequently, a liquid injection cylinder (cylinder with both ends open) having the capacity of 200 mL and having an injection hole of the inner diameter of 3 cm was placed at the center of the main surface of the absorbent article. Subsequently, 160 mL of physiological saline colored with a small amount of Blue No. 1 and adjusted to 25 ± 1°C was injected into the cylinder at one time. Using a stopwatch, the time until physiological saline completely disappeared from the inside of the cylinder was measured, and this time was obtained as the permeation time [seconds]. The results are shown in Table 1.

<Measurement of quick-drying property>

Physiological saline was supplied to the absorbent article in the same manner as the measurement of the above-mentioned permeation time, and then the cylinder was removed. Subsequently, after one minute from the start of supply of physiological saline, 80 sheets of filter papers (75 g in total) of 10 cm × 10 cm having the basis weight of 94 g/m² were piled up and placed as absorbent members in the vicinity of the liquid supply position of the absorbent article. In addition, a weight (bottom surface: 10 cm × 10 cm, mass: 2 kg) was placed on the filter papers. After loading with the weight for 1 minute, the mass of the filter papers was measured, and the amount of increase in mass was obtained as quick-drying property [g]. The value of the quick-drying property is preferably small. The results are shown in Table 1.

[Table 1]

| | Water absorption rate [second] | | Water retention amount [g/g] | Permeation time [second] | Quick-drying property [g] |
|---|---|---|---|---|---|
| | Low-speed flow Vortex method | Vortex method in the related art | | | |
| Example 1 | 37 | 40 | 30 | 41 | 12 |
| Example 2 | 39 | 36 | 35 | 41 | 11 |
| Example 3 | 41 | 45 | 34 | 41 | 10 |
| Example 4 | 32 | 34 | 42 | 42 | 12 |
| Example 5 | 36 | 40 | 45 | 42 | 14 |
| Example 6 | 39 | 50 | 45 | 43 | 14 |
| Example 7 | 48 | 37 | 41 | 45 | 20 |
| Comparative Example 1 | 53 | 44 | 50 | 53 | 19 |
| Comparative Example 2 | 54 | 48 | 44 | 50 | 20 |
| Comparative Example 3 | 56 | 43 | 55 | 52 | 21 |
| Comparative Example 4 | 5 | 4 | 37 | 71 | Unable to measure |

According to Table 1, it is confirmed that, even in a case where the water-absorbent resin particles have a better water absorption rate based on the Vortex method at 600 rpm in the related art, when the water-absorbent resin particles are used for an absorbent article, there is a case where it is difficult to obtain a better permeation rate. In addition, it is confirmed that adjusting the water absorption rate based on the low-speed flow Vortex method at 300 rpm is effective in obtaining an absorbent article having a better permeation rate.

**Reference Signs List**

10: absorber, 10a: water-absorbent resin particles, 10b: fiber layer, 20a, 20b: core wrap, 30: liquid permeable sheet, 40: liquid impermeable sheet, 100: absorbent article.

**Claims**

1. Water-absorbent resin particles comprising:

    a crosslinking polymer comprising a structural unit derived from an ethylenically unsaturated monomer,
    wherein the ethylenically unsaturated monomer comprises at least one compound selected from the group consisting of (meth)acrylic acid and a salt thereof,
    a ratio of the (meth)acrylic acid and the salt thereof is 70 to 100 mol% with respect to a total amount of monomers for obtaining the water-absorbent resin particles,
    a water retention amount of physiological saline is 32 to 80 g/g,
    a water absorption rate of physiological saline based on Vortex method at 300 rpm is 35 to 50 seconds, and
    a water absorption rate of physiological saline based on Vortex method at 600 rpm is 40 to 60 seconds.

2. An absorber comprising:
    the water-absorbent resin particles according to claim 1.

3. The absorber according to claim 2,
    wherein a content of the water-absorbent resin particles is 100 to 1000 g per 1 $m^2$ of the absorber.

4. An absorbent article comprising:
    the absorber according to claim 2 or 3.

5. The absorbent article according to claim 4, which is a diaper.

EP 3 896 097 A1

**Fig.1**

## INTERNATIONAL SEARCH REPORT

| International application No. |
| --- |
| PCT/JP2019/048820 |

A. CLASSIFICATION OF SUBJECT MATTER
C08F 20/06(2006.01)i; C08J 3/12(2006.01)i; A61F 13/53(2006.01)i; B01J
20/26(2006.01)i; B01J 20/28(2006. 01) i
FI: C08J3/12 Z CEY; C08F20/06; A61F13/53 300; B01J20/26 D; B01J20/28 Z
According to International Patent Classification (IPC) or to both national classification and IPC

B. FIELDS SEARCHED

Minimum documentation searched (classification system followed by classification symbols)
C08J3/00-3/28; C08F6/00-246/00,301/00; A61L15/16-15/64; A61F13/15-13/84;
B01J20/26

Documentation searched other than minimum documentation to the extent that such documents are included in the fields searched
Published examined utility model applications of Japan        1922–1996
Published unexamined utility model applications of Japan      1971–2020
Registered utility model specifications of Japan              1996–2020
Published registered utility model applications of Japan      1994–2020

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X | JP 2014-79323 A (LIVEDO CORP.) 08.05.2014 (2014-05-08) claims, paragraphs [0020], [0025], [0026], [0114], examples, in particular, water absorbent resin power 3, 4, examples 3, 4, tables 1-3 | 1-5 |
| X | JP 2014-79324 A (LIVEDO CORP.) 08.05.2014 (2014-05-08) claims, paragraphs [0019], [0023], [0024], [0112], examples, in particular, water absorbent resin power 3, 4, examples 3, 4, tables 1-3 | 1-5 |
| X | WO 2018/147317 A1 (SDP GLOBAL CO., LTD.) 16.08.2018 (2018-08-16) claims, paragraphs [0096], [0097], [0099], [0105], [0113], examples, in particular, example 6, table 1, paragraph [0138] | 1-5 |
| A | JP 7-88171 A (SANYO CHEMICAL INDUSTRIES, LTD.) 04.04.1995 (1995-04-04) entire text | 1-5 |

☒ Further documents are listed in the continuation of Box C.　　☒ See patent family annex.

| | | |
| --- | --- | --- |
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| 03 March 2020 (03.03.2020) | 17 March 2020 (17.03.2020) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

| International application No. |
| --- |
| PCT/JP2019/048820 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | WO 2015/129917 A1 (NIPPON SHOKUBAI CO., LTD.) 03.09.2015 (2015-09-03) entire text | 1-5 |
| A | WO 2016/052537 A1 (NIPPON SHOKUBAI CO., LTD.) 07.04.2016 (2016-04-07) entire text | 1-5 |
| A | WO 2016/158975 A1 (NIPPON SHOKUBAI CO., LTD.) 06.10.2016 (2016-10-06) entire text | 1-5 |
| A | WO 2018/147600 A1 (LG CHEM, LTD.) 16.08.2018 (2018-08-16) entire text | 1-5 |
| A | JP 2003-88552 A (SUMITOMO SEIKA CHEMICALS CO., LTD.) 25.03.2003 (2003-03-25) entire text | 1-5 |
| A | WO 2018/181565 A1 (SUMITOMO SEIKA CHEMICALS CO., LTD.) 04.10.2018 (2018-10-04) entire text | 1-5 |

Form PCT/ISA/210 (continuation of second sheet) (January 2015)

| INTERNATIONAL SEARCH REPORT | International application No. |
|---|---|
| Information on patent family members | PCT/JP2019/048820 |

| Patent Documents referred in the Report | Publication Date | Patent Family | Publication Date |
|---|---|---|---|
| JP 2014-79323 A | 08 May 2014 | US 2015/0297424 A1 claims, paragraphs [0035], [0040], [0041], [0138], examples, in particular, examples 3, 4, tables 1-3 WO 2014/061378 A1 EP 2907492 A1 TW 201420089 A CN 104736115 A | |
| JP 2014-79324 A | 08 May 2014 | US 2016/0199527 A1 claims, paragraphs [0037], [0041], [0042], [0139], examples, in particular, examples 3, 4, tables 1-3 WO 2014/061379 A1 EP 2907493 A1 TW 201420088 A CN 104736116 A | |
| WO 2018/147317 A1 | 16 Aug. 2018 | (Family: none) | |
| JP 7-88171 A | 04 Apr. 1995 | EP 629411 A1 US 2017/0014801 A1 EP 3112022 A1 CN 106029220 A KR 10-2016-0127742 A | |
| WO 2015/129917 A1 | 03 Sep. 2015 | US 2017/0216817 A1 EP 3202823 A1 CN 106715543 A KR 10-2017-0063818 A | |
| WO 2016/052537 A1 | 07 Apr. 2016 | US 2018/0185820 A1 EP 3279238 A1 CN 107428949 A KR 10-2017-0132799 A | |
| WO 2016/158975 A1 | 06 Oct. 2016 | JP 2019-518839 A US 2019/0217272 A1 EP 3453737 A1 KR 10-2018-0092841 A CN 109415516 A | |
| WO 2018/147600 A1 | 16 Aug. 2018 | JP 2019-518839 A US 2019/0217272 A1 EP 3453737 A1 KR 10-2018-0092841 A CN 109415516 A | |
| JP 2003-88552 A | 25 Mar. 2003 | (Family: none) | |
| WO 2018/181565 A1 | 04 Oct. 2018 | CN 110446726 A KR 10-2019-0127698 A | |

Form PCT/ISA/210 (patent family annex) (January 2015)

**EP 3 896 097 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013132433 A
- JP 2008178667 A